(19) Europäisches Patentamt
European Patent Office
Office européen des brevets

(11) Veröffentlichungsnummer : **0 322 360 B1**

(12) **EUROPÄISCHE PATENTSCHRIFT**

(45) Veröffentlichungstag der Patentschrift :
**27.11.91 Patentblatt 91/48**

(51) Int. Cl.$^5$ : **C07C 323/16**

(21) Anmeldenummer : **88810859.4**

(22) Anmeldetag : **13.12.88**

(54) **Verfahren zur Herstellung von phenolischen Thiocarbonsäureestern.**

Die Akte enthält technische Angaben, die nach dem Eingang der Anmeldung eingereicht wurden und die nicht in dieser Patentschrift enthalten sind.

(30) Priorität : **22.12.87 CH 4987/87**

(43) Veröffentlichungstag der Anmeldung :
**28.06.89 Patentblatt 89/26**

(45) Bekanntmachung des Hinweises auf die Patenterteilung :
**27.11.91 Patentblatt 91/48**

(84) Benannte Vertragsstaaten :
**BE CH DE ES FR GB IT LI**

(56) Entgegenhaltungen :
**EP-A- 0 275 832**
**US-A- 4 623 745**

(73) Patentinhaber : **CIBA-GEIGY AG**
**Klybeckstrasse 141**
**CH-4002 Basel (CH)**

(72) Erfinder : **Stegmann, Werner, Dr.**
**Unter der Sonnhalde 9**
**CH-4410 Liestal (CH)**
Erfinder : **Luisoli, Reto**
**Stutzring 3**
**CH-4434 Hölstein (CH)**

**EP 0 322 360 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Einstufenverfahren zur Herstellung von Thiocarbonsäureester aus einem Phenol durch Umsetzung mit Formaldehyd und einem Merkaptan.

Die Herstellung phenolischer Thiocarbonsäureester in zwei Stufen, entweder über die Mannichbase oder durch Umsetzung von entsprechenden Phenolen mit Merkaptosäure und Formaldehyd und anschliessende Veresterung der erhaltenen Säuren, ist bekannt. Sie wird zum Beispiel in der US-A-3832328 beschrieben. Diese Zweistufenverfahren sind jedoch aufwendig.

Die US-A-3553270 beschreibt ein Einstufenverfahren zur Herstellung von phenolischen Thioäthern durch Umsetzung des entsprechenden Phenols mit Formaldehyd und einem Merkaptan in Gegenwart einer starken Base, wie Trimethylamin oder insbesondere Alkalimetallhydroxide, als Katalysator. Versuche, nach dieser Methode auch phenolische Thiocarbonsäureester herzustellen, sind gescheitert.

In der US-A-4623745 ist ein Einstufenverfahren zur Herstellung von phenolischen Thiocarbonsäureestern aus einem Phenol mit Formaldehyd und einem Merkaptosäureester beschrieben. Als Katalysatoren werden unter anderem $C_2$-$C_{20}$-Dialkylamine erwähnt. Konkret wird Dibutylamin als Katalysator eingesetzt.

Da phenolische Thiocarbonsäureester wertvolle Antioxidantien sind, besteht weiterhin das Bedürfnis nach einer Verfahrensverbesserung. Es wurde nun gefunden, dass phenolische Thiocarbonsäureester in hoher Ausbeute und Reinheit in vorteilhaft kurzen Reaktionszeiten erhalten werden, wenn die Umsetzung in Gegenwart von Mono- oder Dimethylamin oder Mono- oder Diethylamin, vorzugsweise Dimethylamin, erfolgt.

Gegenstand vorliegender Erfindung ist daher ein Verfahren zur Herstellung von Verbindungen der Formel I

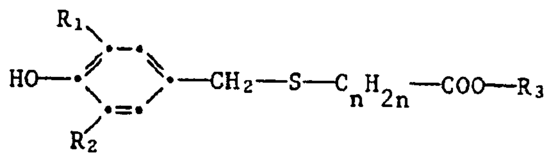

(I),

worin $R_1$ und $R_2$ unabhängig voneinander $C_1$-$C_4$-Alkyl, $R_3$ $C_1$-$C_{20}$-Alkyl oder durch —O— oder —S— unterbrochenes $C_2$-$C_{20}$-Alkyl und n die Zahl 1 oder 2 bedeuten, durch Umsetzung eines Phenols der Formel II

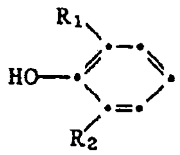

(II)

mit Formaldehyd oder einer unter den Reaktionsbedingungen Formaldehyd freisetzenden Verbindung und mit einem Merkaptan der Formel III

$$HS\text{-}C_nH_{2n}\text{-}COO\text{-}R_3 \quad (III),$$

worin $R_1$, $R_2$, $R_3$ und n die zuvor genannte Bedeutung haben, in Gegenwart einer Base, dadurch gekennzeichnet, dass die Umsetzung bei Ueberdruck und in Gegenwart von Mono- oder Dimethylamin oder Mono- oder Diethylamin als Base erfolgt.

$R_1$ und $R_2$ bedeuten als $C_1$-$C_4$-Alkyl beispielsweise Methyl, Ethyl, n-Propyl, Isopropyl, n-Butyl, sek.-Butyl oder t-Butyl. Bevorzugt ist $R_1$ Methyl und insbesondere t-Butyl. $R_2$ ist bevorzugt t-Butyl.

$R_3$ bedeutet als $C_1$-$C_{20}$-Alkyl zusätzlich zu der Bedeutung von $R_1$ beispielsweise Pentyl, Hexyl, n-Octyl, Oct-3-yl, 2-Ethylhexyl, 1,1,3,3-Tetramethylbutyl, Nonyl, Decyl, Dodecyl, Tridecyl, Hexadecyl, Octadecyl oder Eicosyl. Bevorzugt ist $R_3$ 2-Ethylhexyl, Oct-3-yl oder iso-Tridecyl (Tridecylisomerengemisch).

Beispiele für $R_3$ als durch —O— oder —S— unterbrochenes $C_2$-$C_{20}$-Alkyl sind : Methoxymethyl, 2-Ethoxyethyl, 2-n-Butoxyethyl, 3-n-Butoxypropyl, 2-Octoxyethyl, 2-Hexadecyloxyethyl, 2-Ethoxymethyl, Butoxymethyl, Methoxypropyl, Ethoxypropyl, 3-Thiaheptyl oder 3-Thia-5-methylhexyl.

n ist bevorzugt 1.

Bevorzugt werden nach dem erfindungsgemässen Verfahren Verbindungen der Formel I hergestellt, worin $R_1$ und $R_2$ unabhängig voneinander Methyl oder t-Butyl und $R_3$ $C_6$-$C_{14}$-Alkyl bedeuten, und insbesondere solche, worin $R_1$ und $R_2$ für t-Butyl und n für die Zahl 1 stehen.

Besonders bevorzugt werden Verbindungen der Formel I hergestellt, worin $R_1$ und $R_2$ t-Butyl, n die Zahl 1

und $R_3$ 2-Ethylhexyl, Oct-3-yl oder iso-Tridecyl bedeuten.

Im erfindungsgemässen Verfahren können die Reaktanden Phenol, Formaldehyd und Merkaptan in stöchiometrischen Mengen eingesetzt werden. Mitunter kann es aber von Vorteil sein, wenn der Formaldehyd und/oder das Merkaptan im Ueberschuss eingesetzt werden.

Das erfindungsgemässe Verfahren wird in Gegenwart von Mono- oder Dimethylamin oder Mono- oder Diethylamin als Base durchgeführt. Bevorzugt wird Mono- oder Dimethylamin und insbesondere Dimethylamin eingesetzt.

Die Base kann beispielsweise in einer Menge von 1-50 Mol%, vorzugsweise 10-25 Mol-%, bezogen auf das Merkaptan, eingesetzt werden.

Das erfindungsgemässe Verfahren wird besonders zweckmässig bei einem Ueberdruck von $10^5$ bis $10^6$ Pa durchgeführt. Die Reaktionstemperatur liegt beispielsweise zwischen 80 und 200°C. Ein bevorzugter Temperaturbereich ist 120 und 150°C.

Die Reaktionszeiten können je nach Phenol und Merkaptan schwanken und betragen z.B. 30 Minuten bis 6 Stunden, insbesondere 45 Minuten bis 4 Stunden.

Das erfindungsgemässe Verfahren kann mit oder ohne Lösungsmittel durchgeführt werden. Wird ein Lösungsmittel eingesetzt, so genügen etwa 20 Gew.-% Lösungsmittel, bezogen auf das Endprodukt. Diese Menge stellt eine starke Reduktion der Lösungsmittelmenge gegenüber den üblichen Mengen dar und ist ein Vorteil dieses Verfahrens.

Geeignete Lösungsmittel sind solche, die ein gewisses Lösungsvermögen gegenüber den Reaktanden haben, aber unter den Reaktionsbedingungen im wesentlichen inert sind. Beispiele dafür sind Kohlenwasserstoffe wie Toluol, Xylol, Octan und β-Terpen, Ether wie Dioxan, Diethylether, Dimethylether von Ethylenglykol, Tetrahydrofuran usw. Auch chlorierte Kohlenwasserstoffe, wie Tetrachlorkohlenstoff, Chloroform, Trichlorethan, Perchlorethylene können vorteilhaft eingesetzt werden. Andere geeignete Lösungsmittel sind z.B. Dimethylformamid, Dimethylacetamid oder Dimethylsulfoxid. Auch primäre oder sekundäre Alkohole mit 3 bis 6 C-Atomen, wie Isopropanol, sek.-Butylalkohol, t-Butylalkohol, t-Amylalkohol und Hexylalkohol können für eine erfolgreiche Durchführung des erfindungsgemässen Verfahrens empfohlen werden.

Bevorzugt wird jedoch das erfindungsgemässe Verfahren in Abwesenheit eines Lösungsmittels durchgeführt.

Für die erfindungsgemässe Umsetzung wird Formaldehyd oder eine letzteres unter den Reaktionsbedingungen abgebende Verbindung, wie z.B. Paraformaldehyd oder Hexamethylentetramin, eingesetzt. Bevorzugt wird Formaldehyd und insbesondere Paraformaldehyd verwendet.

Nach dem Abkühlen des Reaktionsgemisches kann das Endprodukt beispielsweise auf dem destillativen Wege gewonnen und gegebenenfalls gereinigt werden.

Es ist jedoch ein weiterer Vorteil des erfindungsgemässen Verfahrens, dass die Endprodukte in einer Reinheit anfallen, die für viele Zwecke ihre direkte Weiterverwendung erlaubt. Ist eine Reinigung des Endproduktes trotzdem erwünscht, so wird die Destillation bevorzugt auf einer Kurzwegdestillationsanlage, vorzugsweise bei einem Druck von 0,5-5 Pa, durchgeführt.

Die als Ausgangsstoffe dienenden Phenole und Merkaptane sind bekannte Verbindungen und sind teilweise im Handel erhältlich oder können nach bekannten Methoden hergestellt werden.

Die nach dem erfindungsgemässen Verfahren hergestellten Verbindungen der Formel I sind bekannte Verbindungen und können als Stabilisatoren für organisches Material gegen dessen Schädigung durch Einwirkung von Sauerstoff, Wärme, Licht oder energiereiche Strahlung verwendet werden.

Bevorzugt ist die Verwendung der Verbindungen als Antioxidantien in organischen Polymeren und in Elastomeren, oder die Verwendung in Mineralölen oder synthetischen Schmierstoffen, wie z.B. in der EP-A-0059168 beschrieben.

Die folgenden Beispiele erläutern die Erfindung weiter. Teile und Prozente beziehen sich darin auf das Gewicht, sofern nichts anderes angegeben ist.

Beispiel 1 : Herstellung von 3,5-Di-t-butyl-4-hydroxybenzyl-merkaptoessigsäure-2'-ethylhexylester

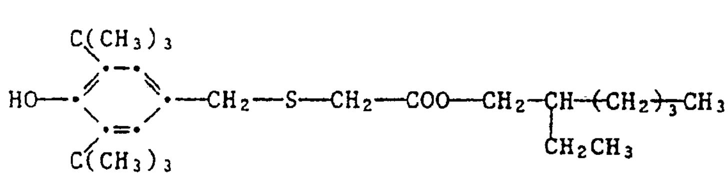

Es werden in einer Apparatur, bestehend aus einem 750 ml Reaktionsgefäss (bis $3 \cdot 10^5$ Pa zugelassen),

welches mit Rührer, Innenthermometer, Stickstoffbegasung und Gaseinleitungsrohr sowie mit Destillationsaufsatz mit Kühler und Vorlage mit Vakuumanschluss versehen ist, nacheinander 206,3 g (1,0 Mol) 2,6-Di-t-butylphenol, 36,0 g (1,2 Mol) 100%-iges Paraformaldehyd und 204,0 g (1,0 Mol) Thioglykolsäure-2-ethylhexylester bei Raumtemperatur vorgelegt. Die Suspension wird mit Stickstoff inertisiert und anschliessend bei mässigem Rühren auf 2000 Pa evakuiert und das Reaktionsgefäss geschlossen. Anschliessend wird durch das Gaseinleitungsrohr 4,5 g (0,1 Mol) Dimethylamin als Gas innert 10 Minuten in die Suspension eingeleitet. Dabei ist eine leichte Exothermie zu beobachten, die Innentemperatur steigt um ca. 5°C und das Vakuum im Reaktionsgefäss beträgt etwa $1,2 \cdot 10^4$ Pa.

Die helle, dünnflüssige Suspension wird auf 130°C aufgeheizt und 4-5 Stunden lang bei dieser Temperatur gerührt. Der Druck steigt dabei auf $2,5 \cdot 10^5$ Pa. Die Suspension geht in eine klare orange-gelbe Schmelze über, die gegen Ende der Reaktion durch das entstandene Wasser stark trübe wird. Das Reaktionsgemisch wird auf 70°C abgekühlt. Bei dieser Temperatur wird durch Anlegen eines Vakuums von 2000 Pa ein Gemisch von Dimethylamin, Wasser und etwas überschüssigem Paraformaldehyd abdestilliert, bis bei 100°C und 2000 Pa die Destillation beendet ist.

Man erhält 413,5 g (98% der Theorie) des Endproduktes mit einem Brechungsindex $n_D^{20}$ von 1,5140.

Beispiel 2 :

Man geht wie im Beispiel 1 vor, jedoch unter Verwendung von 146,0 g (2,0 Mol) Dimethylformamid als Lösungsmittel. Die Suspension wird bei 125°C während 45 Minuten gerührt. Der Druck steigt dabei von ca. $1,2 \cdot 10^4$ Pa auf $1,5 \cdot 10^5$ Pa.

Man erhält 435 g (99% der Theorie) der gewünschten Endproduktes mit einem Brechungsindex $n_D^{20}$ von 1,5120.

Beispiel 3 : Herstellung von 3,5-Di-t-butyl-4-hydroxybenzyl-merkaptoessigsäure-iso-tridecylester

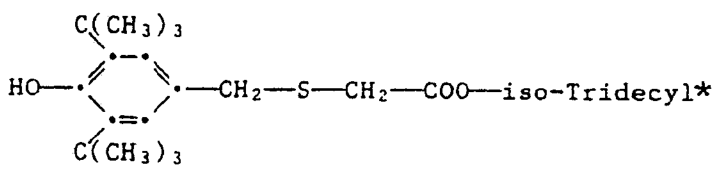

* Isomerengemisch von Tridecanolen

Es werden in einer Apparatur, wie in Beispiel 1 beschrieben, nacheinander 164,8 g (0,8 Mol) 2,6-Di-t-butylphenol, 219,6 g (0,8 Mol) Thioglykolsäure-iso-tridecylester und 26,4 g (0,88 Mol) 100%-iges Paraformaldehyd bei Raumtemperatur vorgelegt.

Die Suspension wird mit Stickstoff inertisiert und anschliessend bei mässigem Rühren auf 2000 Pa evakuiert und das Reaktionsgefäss geschlossen. Anschliessend wird durch das Gaseinleitungsrohr 9,3 g (0,206 Mol) Dimethylamin als Gas innert 10 Minuten in die Suspension eingeleitet. Dabei ist eine deutliche Exothermie zu beobachten, die Innentemperatur steigt um ca. 5-10°C und das Vakuum im Reaktionsgefäss beträgt ca. $2,5 \cdot 10^4$ Pa.

Die helle, dünnflüssige Suspension wird auf 140°C aufgeheizt und 4-5 Stunden lang bei dieser Temperatur gerührt. Der Druck steigt dabei auf $2,3 \cdot 10^5$ Pa. Die Suspension geht in eine klare, gelbbraune Schmelze über, die gegen Ende der Reaktion durch das entstandene Wasser stark trübe wird. Das Reaktionsgemisch wird auf 70°C abgekühlt. Bei dieser Temperatur werden 10,0 g Fluid Cracking Catalyst (FCC) als Absorber zugegeben und durch Anlegen eines Vakuums von 2000 Pa ein Gemisch von Dimethylamin, Wasser und etwas überschüssigem Paraformaldehyd abdestilliert, bis bei 100°C die Destillation beendet ist. Durch Klärfiltration bei 100°C wird die Schmelze abschliessend vom Absorber befreit.

Man erhält 375 g (95% der Theorie) des Endproduktes mit einem Brechungsindex $n_D^{20}$ von 1,5063.

Beispiel 4 :

Man geht wie in Beispiel 3 vor, jedoch unter Verwendung von 114,0 g (1,56 Mol) Dimethylformamid als Lösungsmittel und 5,7 g (0,126 Mol) Dimethylamin. Die Suspension wird bei 135-140°C während 1,5 Stunden gerührt. Der Druck steigt dabei auf $1,9 \cdot 10^5$ Pa.

Man erhält 367 g (93% der Theorie) des Endproduktes mit einem Brechungsindex $n_D^{20}$ von 1,5080.

Beispiel 5 : Herstellung von 3,5-Di-t-butyl-4-hydroxybenzyl-merkaptoessigsäure-oct-3-ylester

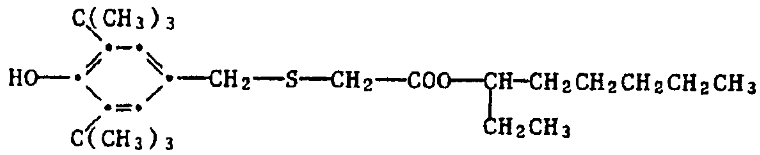

Man geht wie in Beispiel 3 vor, jedoch unter Verwendung von 247,6 g (1,20 Mol) 2,6-Di-t-butylphenol, 244,8 g (1,20 Mol) Thioglykolsäure-oct-3-ylester, 39,6 g (1,32 Mol) 100%-iges Paraformaldehyd und 18,0 g (0,40 Mol) Dimethylamin. Die Suspension wird bei 125°C während 4 Stunden gerührt. Der Druck steigt dabei auf $2,3 \cdot 10^5$ Pa.

Man erhält 482 g (95% der Theorie) des Endproduktes als eine gelbe Flüssigkeit mit einem Brechungsindex $n_D^{20}$ von 1,5130.

Beispiel 6 : Reinigung durch Kurzwegdestillation

In eine Kurzwegdestillationsapparatur (Glas 0,04 $m^2$) wird bei einer Manteltemperatur von 160°C, einer Kühlertemperatur von 27°C, einer Wischergeschwindigkeit von 250 Umdrehungen pro Minute und einem Druck von 0,5 Pa das Rohprodukt aus den Beispielen 1-5, das jeweils auf 70°C vorgeheizt ist, mit einer Dosiergeschwindigkeit von 450 g pro Stunde eingespeist.

Man erhält jeweils ein klares hellgelbes bis gelbes Produkt in einer Ausbeute von ca. 93%, bezogen auf die Menge des eingesetzten Rohproduktes.

Beispiel 7 : Herstellung von 3,5-Di-t-butyl-4-hydroxybenzyl-merkaptoessigsäure-3',4'-Dimethvl-hex-1'-ylester

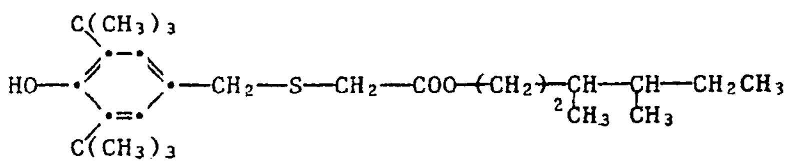

Man geht wie in Beispiel 1 vor, jedoch unter Verwendung von 247,6 g (1,2 Mol) 2,6-Di-butylphenol, 244,8 g (1,2 Mol) Thioglykolsäure-iso-octylester (Isomerengemisch), 39,6 g (1,32 Mol) Paraformaldehyd und 18,0 g (0,4 Mol) Dimethylamin. Dabei ist ein mit Beispiel 1 identischer Reaktionsverlauf zu beobachten. Der Druck steigt während der Reaktion auf $2,3 \cdot 10^5$ Pa.

Man erhält 472 g (93% der Theorie) des Endproduktes mit einem Brechungsindex $n_D^{20}$ von 1,5145.

Dieses Produkt lässt sich leicht durch Destillation in einem Dünnschichtverdampfer (Kopftemperatur : 240°C bei 5 mbar) weiter reinigen.

Man erhält so das Endprodukt mit einer Reinheit von über 97% ; die Dichte beträgt 1,0043 g/ml bei einer Viskosität (bei 40°C) von 197-198 mPa · s.

**Patentansprüche**

1. Verfahren zur Herstellung von Verbindungen der Formel I

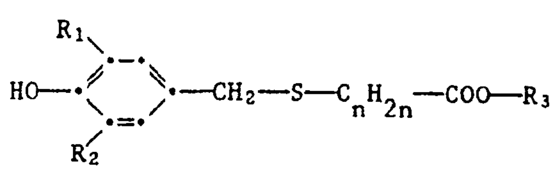

(I),

worin $R_1$ und $R_2$ unabhängig voneinander $C_1$-$C_4$-Alkyl, $R_3$ $C_1$-$C_{20}$-Alkyl oder durch —O— oder —S— unterbrochenes $C_2$-$C_{20}$-Alkyl und n die Zahl 1 oder 2 bedeuten, durch Umsetzung eines Phenols der Formel II

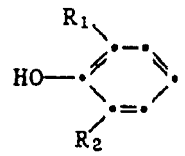

(II)

mit Formaldehyd oder einer unter den Reaktionsbedingungen Formaldehyd freisetzenden Verbindung und mit einem Merkaptan der Formel III

$$HS\text{-}C_nH_{2n}\text{-}COO\text{-}R_3 \quad \text{(III)},$$

worin $R_1$, $R_2$, $R_3$ und n die zuvor genannte Bedeutung haben, in Gegenwart einer Base, dadurch gekennzeichnet, dass die Umsetzung bei Ueberdruck und in Gegenwart von Mono- oder Dimethylamin oder Mono- oder Diethylamin als Base erfolgt.

2. Verfahren nach Anspruch 1, dadurch gekennzeichnet, dass man 1-50 Mol-% der Base, bezogen auf das Merkaptan, einsetzt.

3. Verfahren gemäss Anspruch 1, dadurch gekennzeichnet, dass die Umsetzung in Abwesenheit eines Lösungsmittels erfolgt.

4. Verfahrens gemäss Anspruch 1, dadurch gekennzeichnet, dass ein Ueberdruck von $10^5$ bis $10^6$ Pa angewendet wird.

5. Verfahren gemäss Anspruch 1, dadurch gekennzeichnet, dass die Reaktionstemperatur 120-150°C beträgt.

6. Verfahren nach Anspruch 1, dadurch gekennzeichnet, dass man das Rohprodukt der Formel I durch Kurzwegdestillation reinigt.

7. Verfahren nach Anspruch 1 zur Herstellung von Verbindungen der Formel I, worin $R_1$ und $R_2$ unabhängig voneinander Methyl oder t-Butyl und $R_3$ $C_6$-$C_{14}$-Alkyl bedeuten.

8. Verfahren nach Anspruch 7 zur Herstellung von Verbindungen der Formel I, worin $R_1$ und $R_2$ t-Butyl und n die Zahl 1 bedeuten.

9. Verfahren nach Anspruch 8 zur Herstellung von Verbindungen der Formel I, worin $R_3$ 2-Ethylhexyl, Oct-3-yl oder iso-Tridecyl bedeutet.

10. Verfahren nach Anspruch 1, dadurch gekennzeichnet, dass die Base Dimethylamin ist.

## Claims

1. A process for the preparation of a compound of formula I

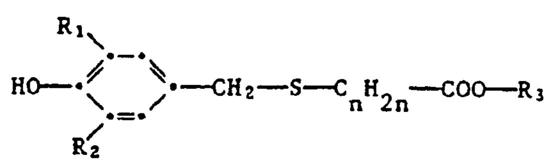

(I),

in which $R_1$ and $R_2$ are each independently of the other $C_1$-$C_4$alkyl, $R_3$ is $C_1$-$C_{20}$alkyl or $C_2$-$C_{20}$alkyl which is interrupted by —O— or —S—, and n is the number 1 or 2, by reacting a phenol of formula II

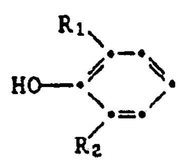

(II),

with formaldehyde or a compound that releases formaldehyde under the reaction conditions, and with a mercaptan of formula III

$$HS\text{-}C_nH_{2n}\text{-}COO\text{-}R_3 \quad \text{(III)},$$

in which $R_1$, $R_2$, $R_3$ and n are as defined above, in the presence of a base, which process comprises carrying

out the reaction under excess pressure and in the presence of mono- or dimethylamine or mono- or diethylamine as base.

2. A process according to claim 1, wherein 1-50 mol% of the base is used, based on the mercaptan.

3. A process according to claim 1, wherein the reaction is carried out in the absence of a solvent.

4. A process according to claim 1, wherein an excess pressure of $10^5$ to $10^6$ Pa is applied.

5. A process according to claim 1, wherein the reaction temperature is in the range from 120 to 150°C.

6. A process according to claim 1, wherein the crude product of formula I is purified by flash distillation.

7. A process according to claim 1 for the preparation of a compound of formula I, in which $R_1$ and $R_2$ are each independently of the other methyl or tert-butyl and $R_3$ is $C_8$-$C_{14}$alkyl.

8. A process according to claim 7 for the preparation of a compound of formula I, in which $R_1$ and $R_2$ are tert-butyl and n is the number 1.

9. A process according to claim 8 for the preparation of a compound of formula I, in which $R_3$ is 2-ethylhexyl, oct-3-yl or isotridecyl.

10. A process according to claim 1, wherein the base is dimethylamine.

**Revendications**

1. Procédé pour préparer des composés répondant à la formule I :

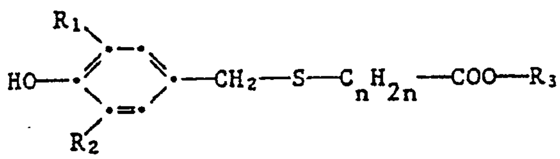

(I),

dans laquelle $R_1$ et $R_2$ représentent chacun, indépendamment l'un de l'autre, un alkyle en $C_1$-$C_4$, $R_3$ représente un alkyle en $C_1$-$C_{20}$ ou un alkyle en $C_2$-$C_{20}$ interrompu par —O— ou —S—, et n désigne un nombre égal à 1 ou à 2, par réaction d'un phénol de formule II :

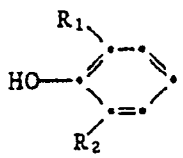

(II)

avec le formaldéhyde ou un composé libérant du formaldéhyde dans les conditions de la réaction et avec un mercaptan répondant à la formule III :

$$HS\text{-}C_nH_{2n}\text{-}COO\text{-}R_3 \quad (III),$$

dans laquelle, $R_1$, $R_2$, $R_3$ et n ont les significations qui leur ont été attribuées ci-dessus, en présence d'une base, procédé caractérisé en ce qu'on effectue la réaction sous pression et en présence de mono- ou de diméthylamine ou de mono- ou de diéthylamine comme base.

2. Procédé selon la revendication 1 caractérisé en ce qu'on utilise de 1 à 50% en moles de la base par rapport au mercaptan.

3. Procédé selon la revendication 1 caractérisé en ce qu'on effectue la réaction sans solvant.

4. Procédé selon la revendication 1 caractérisé en ce que l'on opère sous une surpression de $10^5$ à $10^6$ Pa.

5. Procédé selon la revendication 1 caractérisé en ce que la température réactionnelle est de 120 à 150°C.

6. Procédé selon la revendication 1, caractérisé en ce qu'on purifie le produit brut de formule I par distillation moléculaire.

7. Procédé selon la revendication 1 pour la préparation de composés de formule I dans lesquels $R_1$ et $R_2$ représentent chacun, indépendamment l'un de l'autre, un méthyle ou un ter-butyle, et $R_3$ représente un alkyle en $C_6$-$C_{14}$.

8. Procédé selon la revendication 7 pour la préparation de composés de formule I dans lesquels $R_1$ et $R_2$ représentent chacun un radical ter-butyle et n est égal à 1.

9. Procédé selon la revendication 8 pour la préparation de composés de formule I dans lesquels $R_3$ repré-

sente un radical éthyl-2 hexyle, octyle-3 ou isotridécyle.

10. Procédé selon la revendication 1 caractérisé en ce que la base est la dimétylamine.